# DEMANDE DE BREVET EUROPEEN

(11) **EP 1 502 573 A1**
(43) Date de publication de la demande: **02.02.2005**
(21) Numéro de dépôt: 04291719.5
(22) Date de dépôt: 07.07.2004
(51) Int. Cl.: A61K 7/02

(54) **Produit cosmétique bicouche ses utilisations et kit de maquillage contenant ce produit**

(30) Priorité: 01.08.2003 FR 0309563
(71) Demandeur: L'OREAL, 75008 Paris (FR)
(72) Inventeur: Ricard, Audrey, 75012 Paris (FR); Khachikian, Hélène, 94140 Alfortville (FR); Lemann, Patricia, 94000 Creteil (FR); Girier Dufournier, Franck, 75011 Paris (FR)
(74) Mandataire: Boulard, Denis

(57) **Abrégé**

L'invention se rapporte à un produit cosmétique de maquillage et/ou de soin contenant une première et une seconde compositions, la première composition comprenant un premier milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable.

L'invention se rapporte également à procédé de maquillage ainsi qu'à un kit de maquillage contenant ledit produit.

## Description

La présente invention se rapporte à un nouveau produit cosmétique comprenant au moins deux compositions qui peuvent être appliquées successivement sur la peau aussi bien du visage que du corps humain, sur les paupières inférieures et supérieures des êtres humains, sur les lèvres et sur les phanères comme les ongles, les sourcils, les cils ou les cheveux, ainsi qu'un procédé de maquillage bicouche du visage et du corps humain.

Chaque composition peut être une poudre libre ou compactée, un fond de teint, un fard à joues ou à paupières, un produit anti-cerne, un blush, un rouge à lèvres, un baume à lèvres, un brillant à lèvres, un crayon à lèvres ou à yeux, un mascara, un eye-liner, un vernis à ongles ou encore un produit de maquillage du corps ou de coloration de la peau.

La première composition est destinée à diminuer ou supprimer les défauts de la peau par un effet optique. Elle permet de masquer visuellement les imperfections de surface de la peau, par exemple les rides, les pores, des taches, une texture irrégulière, des différences de ton, des boutons. Elle forme en outre un film translucide sur la peau et laisse ainsi un aspect naturel à la peau tout en améliorant son uniformité et sa luminosité.

La première composition de l'invention ne laisse pas de traces visibles de couleur blanche ou teintée sur la peau. Le consommateur n'a pas besoin de choisir une teinte de produit la plus proche de sa carnation car la composition de l'invention s'adapte à n'importe quel type de peau et procure un effet visuel immédiat d'amélioration de l'uniformité de la peau en masquent les défauts.

La première composition permet également de modifier la perception visuelle du volume de la partie du corps sur laquelle elles sont appliquées.

La première composition peut être utilisée comme base préparatrice pour lisser la peau, notamment les lèvres et leur contour, par effet optique. Associée à la deuxième composition de l'invention, elle améliore la tenue et diminue la migration de cette deuxième composition.

En outre, la sélection d'une deuxième composition brillante et modifiant la perception du volume de la partie du corps sur laquelle la composition est appliquée, permet d'obtenir un produit cosmétique, en particulier un produit de maquillage, de bonne tenue, brillant, confortable, ne migrant pas, dont la tenue sont améliorées.

La présente invention a pour but de proposer un produit de maquillage et/ou de soin de tenue améliorée et dont la migration est diminuée. Ce produit de maquillage masque les imperfections de la peau, et notamment des lèvres et du contour des lèvres. La première composition permet en effet d'améliorer la tenue et de diminuer la migration de la deuxième composition.

La présente invention a également pour but de proposer un produit de maquillage à la fois brillant et de bonne tenue et non migrant, ce qui est difficile à obtenir.

Une des techniques connues pour augmenter la brillance d'un produit de maquillage ou de soin est d'augmenter la proportion de la phase huileuse au détriment de la phase particulaire, cette dernière devant être par ailleurs la mieux dispersée possible. En effet, une mauvaise dispersion des particules pigmentaires peut conduire à un film non homogène sur les lèvres, conférant un aspect peu esthétique. De plus une trop faible quantité de pigments conduit souvent à un film peu couvrant. En outre, la brillance de la composition a tendance à décroître dans le temps, notamment à cause de la mauvaise tenue du film dans le temps.

Il est par ailleurs connu d'épaissir les huiles avec des épaississants polymériques et notamment des polyoléfines. Malheureusement, ces épaississants d'huiles connus doivent être utilisés en grande quantité pour obtenir un épaississement efficace. Or, une trop grande quantité d'épaississant confère à la composition, lorsque cette dernière est destinée au domaine cosmétique, des propriétés cosmétiques insuffisantes, et notamment un toucher collant et un manque de glissant, ces inconvénients pouvant être très gênants, voire rédhibitoires.

Le produit de l'invention permet de limiter, après dépôt sur la peau ou les lèvres, la migration de cette phase dans les rides et ridules, ce qui est particulièrement recherché pour un rouge à lèvres. En effet, une migration importante d'une phase grasse liquide, chargée de matières colorantes, conduit à un effet inesthétique autour des lèvres, accentuant particulièrement les rides et les ridules. Cette migration est souvent citée par les femmes comme un défaut majeur des rouges à lèvres classiques.

Le produit de l'invention est également couvrant en ce sens qu'il masque les imperfections de la peau, et notamment des lèvres et du contour des lèvres.

Le demandeur a constaté de manière surprenante qu'en associant une première et une seconde composition, la première composition comprenant un premier milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable, on peut obtenir un maquillage bicouche qui ne migre pas, de bonne tenue et/ou confortable à l'application et dans le temps (non desséchant, pas de tiraillement).

En particulier, le produit de l'invention permet l'obtention de dépôts brillants, non migrant, dont la tenue sont améliorées. Ces dépôts ne forment pas de traces blanchâtres sur la peau, sont translucides et permettent de maquiller la peau tout en préservant sa couleur et son aspect naturel. Le produit de l'invention permet ainsi un maquillage homogène et esthétique.

Ces propriétés avantageuses font du produit de l'invention un produit particulièrement approprié pour réaliser des produits de maquillage des lèvres tels que rouges à lèvres, brillants à lèvres, des yeux tels que mascara, eye-liners, fards à paupières, ou de la peau du visage tels que les fonds de teint. Il permet également de proposer des produits de maquillage du corps pour atténuer des cicatrices ou des défauts de la structure de la peau.

L'objet de la présente invention est donc un produit cosmétique, notamment de maquillage et/ou de soin contenant une première et une seconde compositions, la première composition comprenant un premier milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable.

Par produit de maquillage, on entend un produit contenant un agent colorant permettant le dépôt d'une couleur sur une matière kératinique (la peau, les lèvres ou les phanères) tels que des rouges à lèvres, des fards, des eye-liners, des fonds de teint ou des autobronzants, des produits de maquillage semi permanent (tatouage).

Le produit selon l'invention comprend deux (ou plusieurs) compositions physiologiquement acceptables conditionnées séparément ou ensemble dans un même article de conditionnement ou dans deux (ou plusieurs) articles de conditionnement séparés ou distincts.

De préférence, ces compositions sont conditionnées séparément et avantageusement, dans des articles de conditionnement séparés ou distincts.

L'objet de la présente invention est donc en particulier un produit cosmétique de maquillage ou de soin se présentant sous la forme d'un fond de teint, d'un fard à joues ou à paupières, d'un rouge à lèvres, d'un produit ayant notamment des propriétés de soin, d'un eye-liner, d'un produit anti-cernes, ou d'un produit de maquillage du corps (du type tatouage).

L'invention a aussi pour objet un kit de maquillage contenant un produit cosmétique de maquillage ou de soin tel que défini précédemment, dans lequel les différentes compositions sont conditionnées séparément et sont accompagnées de moyens d'application appropriés. Ces moyens peuvent être des pinceaux, des brosses, des stylos, des crayons, des feutres, des plumes, des éponges et /ou des mousses.

La première composition du produit selon l'invention peut constituer une couche de base appliquée sur la matière kératinique et la seconde composition une couche de dessus. Il est toutefois possible d'appliquer sous la première couche une sous couche ayant la constitution ou non de la seconde couche.

Il est aussi possible de déposer une surcouche sur la seconde couche ayant une constitution identique ou non à celle de la première couche. De préférence, le maquillage obtenu est un maquillage bicouche.

En particulier la couche de base est une base de maquillage, un produit correcteur, un fond de teint, un fard, un rouge à lèvres, un eye liner, un produit de maquillage du corps, et la couche du dessus un produit de soin ou de protection, un brillant à lèvres.

L'invention se rapporte aussi à un procédé de maquillage de la peau et/ou des lèvres et/ou des phanères consistant à appliquer sur la peau et/ou les lèvres et/ou les phanères un produit cosmétique de maquillage tel que défini précédemment.

L'invention a encore pour objet un procédé de maquillage ou de soin de la peau et/ou des lèvres et/ou des phanères d'être humain, consistant à appliquer sur la peau, les lèvres et/ou les phanères une première couche d'une première composition comprenant un milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable.

La seconde couche peut former des motifs et peut être appliquée avec un stylo, crayon ou tout autre instrument (éponge, doigt, pinceau, brosse, mousse, plume etc.). Ce maquillage peut aussi être appliqué sur les accessoires de maquillage comme les faux ongles, faux cils, perruques ou encore des pastilles ou des patchs adhérents sur la peau ou les lèvres (du type mouches).

L'invention a aussi pour objet un support maquillé comprenant une première couche d'une première composition comprenant un premier milieu physiologiquement acceptable et présentant un indice de flou (Th-Td)/Th*100 supérieur ou égal à 40%, et un indice de transparence Th supérieur ou égal à 70%, et une seconde couche d'une seconde composition déposée sur tout ou partie de la première couche, comprenant un second milieu physiologiquement acceptable.

Ce support peut être en particulier un postiche tel qu'une perruque, des faux ongles, des faux cils, ou encore des patchs adhérents sur la peau ou les lèvres (du type mouches).

L'invention a enfin pour objet l'utilisation d'un produit cosmétique de maquillage contenant une première et une seconde compositions, la première composition comprenant un premier milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable pour laisser sur la peau et/ou les lèvres et/ou les phanères un dépôt brillant, confortable, de bonne tenue, de bonne tenue de la couleur, de bonne tenue de la brillance, non migrant et/ou qui masquent les imperfections de la peau, des lèvres ou des ongles.

### Première composition

La première composition selon l'invention comprend donc, dans un premier milieu physiologiquement acceptable,

Par «milieu physiologiquement acceptable », on entend un milieu non toxique et susceptible d'être appliqué sur la peau, les phanères ou les lèvres du visage d'êtres humains.

Par « cosmétiquement acceptable », on entend au sens de l'invention une composition d'aspect, d'odeur, de goût et de toucher agréables.

La première composition selon l'invention comprend un premier milieu physiologiquement acceptable et présente un indice de flou (Th-Td)/Th*100 supérieur ou égal à 40%, et un indice de transparence Th supérieur ou égal à 70%.

Une telle composition confère au teint, aux lèvres et plus généralement la peau, une plus grande uniformité visuelle, une plus grande homogénéité, de la transparence et un teint lumineux. Appliquée sur les lèvres, la composition de l'invention permet de lisser les lèvres par effet optique, de masquer les rides et les imperfections des lèvres et du contour des lèvres.

L'indice de flou (Th-Td)/Th*100 peut être mesuré selon le protocole décrit ci après à l'aide d'un spectrophotomètre et d'une sphère d'intégration par exemple placée derrière l'échantillon.
- Th est la transmittance hémisphérique de la composition: elle est définie par le rapport entre l'intensité lumineuse reçue par la composition et l'intensité lumineuse restituée par la composition dans toutes les directions,
- Td est la transmittance directe de la composition: elle est définie par le rapport entre l'intensité lumineuse reçue par la composition et l'intensité lumineuse restituée par la composition dans le même axe.

Selon un mode de réalisation, Th et Td sont mesurées à l'aide d'un spectrophotomètre VARIAN Cary 300 et d'une sphère d'intégration de marque Labsphere placée derrière l'échantillon, selon le protocole suivant. Un film de la composition selon l'invention, d'épaisseur 20 µm, est étalé sur une lame de quartz à creuset, puis placé 5 minutes à 37°C.

Pour mesurer Th, on utilise le spectrophotomètre en mode de transmission diffuse, à une longueur d'onde variant de 400 à 700 nm. On se place en mode transmission %T, à une vitesse de balayage à 240 nm/min, en « double reverse ». On fait une correction de la ligne de base en faisant la mesure d'une lame vide de référence. Ceci donne la valeur maximale de l'intensité qui peut être transmise. On place la lame de quartz contenant le film de composition dans le compartiment de mesure et on mesure Th.

Td est mesurée à l'aide du même spectrophotomètre selon le protocole suivant : on utilise le spectrophotomètre en mode de transmission directe, à une longueur d'onde variant de 400 à 700 nm. On se place en mode transmission %**T**, à une vitesse de balayage 240 nm/min, en mode double. On place une lame de quartz vide dans le compartiment de référence et la lame de quartz contenant l'échantillon dans le compartiment de mesure. On mesure Td.

Les valeurs de Th et Td sont les moyennes de chacune des valeurs spectrales mesurées.

Plus la valeur de (Th-Td)/Th*100 est élevée, meilleur est l'effet flou. Plus la valeur de Td est élevée, plus la composition est transparente.

L'indice de flou est supérieur ou égal à 40%, de préférence supérieur ou égal à 50%.

L'indice de transparence est avantageusement supérieur ou égal à 85%, de préférence supérieur ou égal à 95%.

La première composition contient un composé minéral ou organique apte à conférer à la composition un tel indice de flou et un tel indice de transparence. Ce composé est de préférence en quantité suffisante dans la composition pour conférer à la composition l'indice de flou et l'indice de transparence définis ci-dessus.

Ledit composé peut être choisi par exemple parmi les charges, les organopolysiloxanes et les polymères non siliconés de faible masse moléculaire.

Par charges, il faut comprendre des particules colorées ou blanches, minérales ou organiques, lamellaires ou non lamellaires, solides à température ambiante (25°C) et pression atmosphérique, qui n'interagissent pas chimiquement avec les autres ingrédients de la composition et qui sont en particulier insolubles dans ces ingrédients même lorsque ces ingrédients sont portés à une température supérieure à la température ambiante.

Par charges organiques, on entend des charges constituées d'un unique composé chimique organique ou des charges constituées d'un substrat organique enrobée d'un autre composé organique ou minéral.

Par charges minérales, on entend des charges constituées d'un unique composé chimique minéral ou des charges constituées d'un substrat minéral enrobée d'un autre composé organique ou minéral.

Lesdites charges utilisées pour conférer à la composition de l'invention un indice de flou et un indice de transparence suffisants sont de préférence sous la forme d'une poudre. La granulométrie moyenne des charges est avantageusement inférieure ou égale à 50 microns, de préférence 30 microns, de préférence encore 15 microns, et préférentiellement inférieure ou égale 10 microns, par exemple de l'ordre de 5 ou 3 microns.

Parmi les charges d'origine naturelle ou synthétique, on peut citer notamment les charges telles que le talc, l'amidon, le mica, la silice, les poudres de nylon, les poudres de polyéthylène, la poly-bêta-alanine, les poly(méthacrylate de méthyle).

Lesdits organosiloxanes sont notamment sous forme de gel constitué d'un organopolysiloxane élastomère de structure tridimensionnelle, inclus dans au moins une huile hydrocarbonée et/ou une huile siliconée.

Les organopolysiloxanes élastomères peuvent être choisis parmi les polymères réticulés décrits dans la demande EP-A-0295886.

Selon cette demande, ils sont obtenus par réaction d'addition et de réticulation d'au moins :
(a) un organopolysiloxane ayant au moins deux groupes alcenyls inférieurs par molécule ;
(b) un organopolysiloxane ayant au moins deux atomes d'hydrogène liés à un atome de silicium par molécule ; et
(c) et un catalyseur du type platine.

Les organopolysiloxanes élastomères peuvent être aussi choisis parmi ceux décrits dans le brevet US 5.266.321.

Selon ce brevet, ils sont choisis notamment parmi :
i) les polyorganopolysiloxanes comprenant des motifs R₂SiO et R SiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel que vinyle et où le rapport en poids des motifs R₂SiO sur les motifs RSiO_{1,5} varie de 1/1 à 30/1 ;
ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

La phase grasse associée à l'organopolysiloxane solide élastomère pour former un agent matifiant est constituée d'au moins une huile hydrocarbonée et/ou d'au moins une huile siliconée.

Les huiles hydrocarbonées utilisées en association avec l'organopolysiloxane élastomère peuvent être choisies parmi :
- les huiles d'origine animale telles que le perhydrosqualène ;
- les huiles végétales telles que les triglycérides liquides, par exemple les huiles de tournesol, de maïs, de soja, de courge, de pépins de raisin, de sésame, de noisette, d'abricot, de macadamia, de ricin, les triglycérides des acides caprylique/caprique comme ceux vendus par la société STEARINERIES DUBOIS ou ceux vendus sous les dénominations MIGLYOL 810, 812 et 818 par la société DYNAMIT NOBEL ;
- les huiles de formule R₉COOR₁₀ dans laquelle R₉ représente le reste d'un acide gras supérieur comportant de 7 à 19 atomes de carbone et R₁₀ représente une chaîne hydrocarbonée ramifiée contenant de 3 à 20 a tomes de carbone, par exemple, l'huile de Purcellin ;
- les hydrocarbures linéaires ou ramifiés, d'origine minérale ou synthétique tels que les huiles de paraffine et leurs dérivés, l'isoparaffine, l'isohexadécane, le squalane et équivalents tel que les polyisobutènes, les polydécènes ;
- des esters et les éthers de synthèse ;
- leurs mélanges.

Les huiles siliconées qui peuvent être utilisées en association avec l'organopolysiloxane élastomère sont choisies de préférence parmi les polysiloxanes linéaires liquides ou pâteux à température ambiante tels que méthylpolysiloxane, méthylphénylpolysiloxane, éthylpolysiloxane, éthylméthylpolysiloxane, éthylphénylpolysiloxane, hydroxyméthylpolysiloxane, alkylpolydiméthylsiloxane et les polysiloxanes cycliques tels que octaméthylcyclopentasiloxane, décaméthylcyclopentasiloxane; ou leurs mélanges.

De façon préférentielle, l'organopolysiloxane est présent dans le mélange organopolysiloxane/phase grasse pour former l'agent matifiant sous forme d'un gel homogène dans une concentration allant de 3 à 80 % en poids.

On peut utiliser les organopolysiloxanes connus sous les références commerciales KSG (KSG 6, 16, 17, 18) de la société Shin Etsu, Tréfils de la société Dow Corning ou Gransils pour la société Grand Industrie.

Lesdits polymères non siliconés de faible masse moléculaire sont notamment des polymères amide, uréthanne ou urée. Ces polymères présentent avantageusement des chaînes pendantes et/ou terminales alkyles ou alkylènes, comprenant de préférence de 8 à 60 atomes de carbone, de préférence encore de 12 à 40 atomes de carbone.
Leur masse moléculaire moyenne en poids est de préférence comprise entre 200 et 1000 g/mol.

De préférence, le composé polymérique est un polyamide. On donnera comme exemple un polyamide résultant d e l'amidification d'un triglycéride p ar u ne d iamine éventuellement e n présence d'un monoacide carboxylique en C₁₂ à C₄₀. Le triglycéride est en particulier un triglycéride d'acide gras hydroxylé ayant de 12 à 30 atomes de carbone comme l'acide ricinoléïque (ou huile de ricin) et l'acide monocarboxylique est notamment l'acide 12-hydroxystéarique. La diamine est en particulier l'éthylène diamine.

Selon un de ses aspects, la première composition comprend au moins une phase grasse contenant au moins une huile volatile.

Par "huile volatile", on entend tout milieu non aqueux susceptible de s'évaporer au contact de la peau, à température ambiante (25°C) et pression atmosphérique (760 mm de Hg). Le ou les huiles cosmétiques volatiles, liquides à température ambiante, o nt notamment une pression de vapeur, mesurée à température ambiante et pression atmosphérique, allant de 10⁻³ à 300mm de Hg (0,266 Pa à 40 000 Pa), de préférence de 0,02 mm à 300mm de Hg (2,66 Pa à 40 000 Pa) et mieux allant de 0,1 à 90 mm de Hg (13 Pa à 12 000 Pa).

Selon l'invention, ces huiles volatiles peuvent être des huiles hydrocarbonées, des huiles siliconés comportant éventuellement des groupements alkyle ou alkoxy pendants ou en bout de chaîne siliconée ou un mélange de ces huiles.

De préférence, les huiles volatiles sont des huiles cosmétiques choisies parmi les huiles n'ayant pas de point éclair, les huiles ayant un point éclair allant de 40°C à 100°C, et leurs mélanges, en vue de faciliter leur mise en oeuvre. En outre, ils présentent avantageusement une température d'ébullition à pression atmosphérique inférieure à 220°C et mieux inférieure à 210°C, notamment allant de 110 à 210°C. De préférence, ces huiles volatiles ne sont pas des monoalcools à au moins 7 atomes de carbone.

Comme huile volatile utilisable dans l'invention, on peut citer les huiles de silicones linéaires ou cycliques ayant une viscosité à température ambiante inférieure à 8 cSt et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthylcyclo-tétrasiloxane, le décaméthylcyclopentasiloxane, le dodécaméthylcyclohexasiloxane, l'heptaméthyl hexyltrisiloxane, l'heptaméthyloctyl trisiloxane, l'hexaméthyldisiloxane, l'octaméthyl trisiloxane, le décaméthyl tétrasiloxane, le dodécaméthyl pentasiloxane et leurs mélanges.

Comme autre huile volatile utilisable dans l'invention, on peut citer les huiles volatiles hydrocarbonées ayant de 8 à 16 atomes de carbone et leurs mélanges et notamment les alcanes ramifiés en C₈-C₁₆ comme les isoalcanes (appelés aussi isoparaffines) en C₈-C₁₆, l'isododécane, l'isodécane, l'isohexadécane et par exemple les huiles vendues sous les noms commerciaux d'Isopars' ou de Permetyls, les esters ramifiés en C₈-C₁₆ comme le néo pentanoate d'iso-hexyle et leurs mélanges.

De préférence, on utilise l'isododécane (Permetyls 99 A), les isoparaffines en C₈-C₁₆ comme l'Isopar L, E, G ou H, leurs mélanges, éventuellement associés au décaméthyl tétrasiloxane ou au cyclopentasiloxane.

On peut aussi utiliser des huiles volatiles fluorées.

Ces huiles volatiles représentent notamment de 5 à 80 % du poids total de la composition, et mieux de 10 à 30 %.

Selon un autre aspect, la première composition comprend au moins une charge organique et au moins une charge minérale.

Par charges organiques, on entend des charges constituées d'un unique composé chimique organique ou des charges constituées d'un substrat organique enrobée d'un autre composé organique ou minéral.

Par charges minérales, on entend des charges constituées d'un unique composé chimique minéral ou des charges constituées d'un substrat minéral enrobée d'un autre composé organique ou minéral.

De préférence, la charge organique et la charge minérale de l'invention ont indépendamment l'une de l'autre une granulométrie moyenne de particules inférieure ou égale à 50 microns, de préférence 30 microns, de préférence encore 15 microns, et préférentiellement inférieure ou égale 10 microns, par exemple de l'ordre de 5 ou 3 microns.

On peut ainsi obtenir une composition qui comprend peu de pigments, voire aucun pigment mais qui camoufle bien les micro reliefs et autres imperfections de la peau. Selon un mode réalisation, la composition de l'invention contient des pigments dans une quantité telle qu'ils ne diminuent pas l'indice de flou et l'indice de transparence de la composition. La composition est de préférence exempte de pigments ou de nacres.

La charge minérale est constituée ou comprend au moins un matériau choisi par exemple parmi le talc, le mica, la silice, le kaolin, les microsphères de silice creuses, les microcapsules de verre, les oxydes de titane, les oxydes de fer, les oxydes de zinc, les oxydes de cérium, les oxydes d'aluminium, le sulfate de baryum, les hydroxydes métalliques et leurs mélanges.

La charge minérale peut être constituée d'un substrat de mica enrobé d'alumine, de dioxyde de titane, de silice, d'oxyde d'aluminium et/ou de sulfate de baryum.
La charge peut être également choisie parmi les composites de silice enrobés de TiO₂, les composites de TiO₂ enrobés de silice, les composites silice/oxyde de zinc et leurs mélanges.

Elles peuvent être de forme lamellaire ou non lamellaire.

La première composition comprend avantageusement une charge minérale comprenant du mica, ladite charge ayant une taille moyenne des particules inférieure o u égale à 50, de préférence inférieure à 10 microns, de préférence encore inférieure à 5 microns.

Parmi les charges minérales convenant particulièrement bien à l'invention, on peut citer :
- l'oxyde de titane recouvert de silice tel que Flonac TS 40 C distribué par Eckart,
- des microbilles de silice de granulométrie comprise entre 3 et 12 microns telles que Silica Beads SB 150 fabriquées par Miyoshi ou Sunsphere H de granulométrie comprise entre 3 et 12 microns fabriquées par Asahi Glass,
- des plaquettes de silice telles que Chemiceler distribuées par Sumitomo ou Finesil F-80 également distribuées par Sumitomo, de granulométrie égale à 1,5 micron,
- de la silice enrobée de dioxyde de titane et de silice poreuse, de granulométrie par exemple égale à 0,6 micron, comme le produit ACS-0050510 de Catalysts et Chemicals telle que la proportion silice/dioxyde de titane/silice poreuse est égale à 85/5/10,
- du mica recouvert de sulfate de baryum et d'oxyde de titane, comme le produit Naturaleaf de Merck telle que la proportion mica/sulfate de baryum/oxyde de titane est égale à 66/22/12,
- un complexe de silice et d'oxyde de cérium enrobé de silice amorphe, de granulométrie comprise entre 1 et 10 microns, comme le produit Ceriguard SC,
- un complexe de silice et d'oxyde de titane enrobé de polyhydrogénométhylsiloxane dans les proportions 93/5/2,
- des plaquettes de séricite recouvertes d'oxyde de titane, d'alumine et de silice, comme par exemple le Coverleaf AR-20121 M commercialisé par Catalysts et Chemicals dont la granulométrie est comprise entre 5 et 10 µm et telle que la proportion séricite/oxyde de titane/alumine/silice est égale à 67/5/18/10.
- le talc.

Selon un mode de mis en oeuvre, la première composition comprend une charge minérale choisie parmi les composites d'un silicate inorganique et d'un hydroxyde métallique.

Le silicate inorganique est de préférence du mica naturel ou synthétique. Le mica peut être choisi parmi les micas muscovite, phlogopite, tiotite, sericite, lepidolite, paragonite, les micas synthétiques et leurs mélanges.

L'hydroxyde métallique est un hydroxyde d'un métal choisi parmi Ag, Au, Cu, Al, Ni, Sn, Mg, Cr, Mo, Ti, Pt, Va, Rb, W, Zn, Ge, Te, Se et leurs alliages. On préfère l'hydroxyde d'aluminium comme hydroxyde métallique.

Selon un mode de mise en oeuvre, la charge minérale est un composite constituée de particules de silicate inorganique recouvertes en surface par l'hydroxyde métallique. Le silicate inorganique est avantageusement sous forme lamellaire ou sous forme de plaquettes. Par l'expression « sous forme de plaquettes », on désigne des particules dont le rapport entre la plus grande dimension et l'épaisseur est supérieur ou égal à 5, voire 10 ou mieux 20.

L'hydroxyde métallique est avantageusement sous forme essentiellement sphérique, notamment sous la forme de billes, de préférence de granulométrie comprise entre 1 et 15 microns de préférence de l'ordre de 10 microns.

La proportion massique entre le silicate inorganique et l'hydroxyde métallique est de préférence comprise entre 50/50 et 80/20, de préférence entre 60/40 et 70/30, de préférence égale à environ 65/35.

Les billes d'hydroxyde métallique sont avantageusement des billes d'hydroxyde d'aluminium.

On préfère les plaquettes de mica recouvertes de micro-billes d'hydroxyde d'aluminium dans un rapport pondéral de 60/40 ou de 65/35, notamment le produit Excel Mica JP-1 ou le produit Excel Mica JP-2 commercialisés par Miyoshi.

Selon cet aspect de l'invention, la composition peut contenir en outre de l'amidon éventuellement modifié.

De préférence, la charge minérale est présente dans la composition à une teneur allant avantageusement de 0,1 à 40 %, de préférence encore de 3 à 15%, de préférence encore de 2% à 8%, en poids par rapport au poids total de la composition, de préférence de l'ordre de 5 %.

Parmi les charges organiques convenant à l'invention, on peut citer les poudres de polyamide comme le Nylon®, les poudres de poly-β-alamine, les poudres de polyéthylène, les poudres de polytétrafluoroéthylène, la lauroyl-lysine, l'amidon, les microsphères creuses polymériques telles que celles de chlorure de polyvinylidène/acrylonitrile comme l'Expancel ®, les copolymères d'acide acrylique comme le Polytrap ®, les microbilles de résine de silicone, par exemple le Topspearl.

Parmi les charges organiques, convenant à l'invention, on peut citer les structures multicouches de composés polymériques.

La première composition comprend avantageusement une charge organique comprenant de l'amidon, ladite charge ayant une taille moyenne des particules inférieure ou égale à 50, de préférence inférieure à 10 microns, de préférence encore inférieure à 5 microns.

Selon un mode de réalisation préféré, la charge organique est de l'amidon, en particulier de l'amidon modifié, comme par exemple de l'amidon réticulé par l'anhydride octénylsuccinique commercialisé sous la référence Dry Flo Plus (28-1160) par National Starch.

De préférence, la charge organique est présente dans la composition à une teneur allant avantageusement de 0,1 à 40 %, de préférence encore de 3 à 15%, de préférence encore de 2% à 8%, en poids par rapport au poids total de la composition, de préférence de l'ordre de 5%.

La charge organique et la charge minérale ont de préférence un indice de réfraction supérieur à 1, de préférence allant de 1,25 et 1,9, plus préférentiellement allant de 1,45 et 1,55.

La charge minérale et la charge organique sont de préférence dans un rapport massique compris entre 1 :1 et 1 :3.

Selon un mode mise en oeuvre, la charge minérale est lamellaire lorsque la charge organique est sphérique et vice versa.

La composition selon l'invention contient avantageusement moins de 30% de charges, de préférence moins de 20% de charges.

Selon un autre aspect, la première composition comprend des particules de mica traitées en surface par de l'hydroxyde d'aluminium et présente
- un indice de flou (Th-Td)/Th*100 supérieur ou égal à 40%, et
- un indice de transparence Th supérieur ou égal à 70%
Th étant la transmittance hémisphérique de ladite composition et Td étant la transmittance directe de ladite composition.

Les particules de mica s ont avantageusement en une quantité suffisante pour obtenir un indice de flou et un indice de transparence satisfaisant.

Selon un autre aspect, la première composition comprend des particules de mica enrobées ou non enrobées et des particules d'amidon modifié ou non modifié.

La composition selon l'invention peut comprendre au moins une huile non volatile.

Par huile non volatile, on entend un corps gras liquide à température ambiante (20°C) et qui ne s'évapore pas à cette même température.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment les phényltriméthicones,
- les huiles d'origine végétale ou minérale, telles que l'huile de paraffine, de vaseline, le perhydrosqualène, la lanoline, l'huile d'abricot, l'huile de germes de blé, d'amande douce, de calophyllum, de sésame, de macadamia, de pépins de raisin, de colza, de coprah, d'arachide, de tournesol, de palme, de ricin, d'avocat, de jojoba, d'olive ou de germes de céréales, la fraction liquide du beurre de karité; des esters d'acides gras et de polyol, en particulier les triglycérides liquides, le myristate d'isopropyle, l'huile de mygliol, le néopentanoate d'isostéaryle, le palmitate d'éthyl-2-hexyle, l'huile de ricin, l'acétyl-citrate de tri-butyle; des alcools, en particulier l'octyl-2-dodecanol ; des acétylglycérides ; des octanoates, décanoates ou ricinoléates d'alcools ou de polyalcools ; des triglycérides d'acides gras; des glycérides, les huiles fluorées et perfluorées ;
- les composés amidés, en particulier ceux décrits dans la demande PCT/FR98/01077 comme le N-néopentanoyl-2-octyl-dodécylamine, le N-néopentanoyl-2-butyl-octylamine, le N-(3,5,5-triméthyl-hexanoyl)-2-octyl-dodécylamine, le N-(3,5,5-triméthyl-hexanoyl)-2-butyl-octylamine,
- leurs mélanges.

De p référence, l'huile n on volatile d e la p hase grasse d e la composition selon l'invention comprend une ou plusieurs huiles choisies parmi le polyisobutène hydrogéné, le propionate d'arachidyle, le néopentanoate d'octydodécyle, le polybutène, les diméthicones, l'octyldodécanol, et leurs mélanges.

De préférence, l'huile non volatile représente de 1 à 85%, de préférence encore de 5% à 50%, en poids par rapport au poids total de la composition.

La composition peut comprendre une cire et/ou un pâteux, en particulier lorsqu'elle de présente sous la forme d'un stick.

Par "pâteux" au sens de la présente invention, on entend un composé gras lipophile à changement d'état solide/liquide réversible, présentant à l'état solide une organisation cristalline a nisotrope, et comportant à la température de 23°C une fraction liquide et une fraction solide. Le composé pâteux au sens de l'invention a avantageusement une dureté à 20°C allant de 0,001 à 0,5 MPa, de préférence de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20°C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23°C, sous la forme d'une fraction liquide et d'une fraction solide. En d'autres termes, la température de fusion commençante du composé pâteux est inférieure à 23°C. La fraction liquide du composé pâteux mesurée à 23°C représente 9 à 97% en poids du composé. Cette fraction liquide à 23°C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.
L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.
L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.
L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.
Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi
- la lanoline et ses dérivés
- les composés siliconés polymères ou non
- les composés fluorés polymères ou non
- les polymères vinyliques, notamment:
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, de préférence en C2-C50,
- les esters,
et leurs mélanges.

Le composé pâteux représente de préférence 1 à 99%, mieux 1 à 60%, mieux 2 à 30% et mieux encore 5 à 15% en poids de la composition.

Parmi les cires, on peut notamment citer :
- les cires d'origine animale, végétale, minérale ou synthétique telles que les cires microcristallines, la paraffine, le pétrolatum, la vaseline, l'ozokérite, la cire de Montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25 °C, les ozokérites, les esters gras et les glycérides concrets à 25 °C; les cires de polyéthylène et les cires obtenues par synthèse de Fischer-Tropsch; des huiles hydrogénées concrètes à 25 °C; des lanolines; des esters gras concrets à 25 °C; les cires de silicone; les cires fluorées; leurs mélanges.

La composition selon l'invention peut contenir des charges ne permettant pas de conférer à la composition les propriétés décrites précédemment.

Ces charges peuvent être présentes dans la composition à raison de 0-20 % en poids, de préférence de 2-15 % en poids, par rapport au poids total de la composition. La composition est de préférence exempte de telles charges.

### Seconde composition

Le produit cosmétique de maquillage selon l'invention contient une seconde composition comprenant un second milieu physiologiquement acceptable.

La seconde composition peut être toute composition connue de l'homme du m étier, pour laquelle l'homme du métier souhaite améliorer au moins une propriété cosmétique, en particulier améliorer la tenue, la non migration et/ou le masquage des imperfections des matières kératiniques.

Lorsque le produit cosmétique de l'invention est un produit de maquillage, la seconde composition peut être toute composition cosmétique de maquillage connue de l'homme du métier. En particulier, la seconde composition peut être un rouge à lèvres ou un gloss.

Selon un mode préféré de réalisation de l'invention, le milieu physiologiquement acceptable de la seconde composition comprend une phase liquide non volatile à température ambiante et pression atmosphérique.

Par « phase liquide non volatile », on entend tout milieu susceptible de rester sur la peau ou les lèvres pendant plusieurs heures. Une phase liquide non volatile a en particulier une pression de vapeur à température ambiante et pression atmosphérique, non nulle inférieure à 0,02 mm de Hg (2,66 Pa) et mieux inférieure à 10⁻³ mm de Hg (0,13 Pa).

La phase liquide non volatile de la seconde composition peut être une phase hydrocarbonée liquide, une phase siliconée liquide et/ou une phase fluorée liquide à température ambiante.

Selon un mode réalisation, la deuxième composition cosmétique comporte des nacres, des agents de coloration monocolores, des agents de coloration réléchissants et/ou des agents de coloration goniochromatiques.

Selon un mode réalisation, la deuxième composition cosmétique comporte, dans un milieu physiologiquement acceptable, au moins un agent de coloration goniochromatique et au moins des particules réfléchissantes distinctes de l'agent de coloration goniocromatique.

Les particules réfléchissantes peuvent être choisies dans le groupe constitué par :
- les particules à substrat naturel ou synthétique enrobé au moins partiellement par au moins une couche d'au moins un métal,
- les p articules à s ubstrat s ynthétique e nrobé a u moins partiellement p ar a u moins une couche d'au moins un composé métallique et notamment d'un oxyde métallique,
- les particules formées d'un empilement d'au moins deux couches de matériaux à indices de réfraction différents, l'une au moins de ces couches pouvant être un polymère, et
- les particules d'oxydes métalliques.

Par « agent de coloration goniochromatique », on désigne au sens de la présente invention un agent de coloration permettant d'obtenir, lorsque la composition cosmétique est étalée sur un support, un trajet de couleur dans le plan a*b* de l'espace colorimétrique CIE 1976 qui correspond à une variation Dh de l'angle de teinte h d'au moins 20° lorsque l'on fait varier l'angle d'observation par rapport à la normale entre 0° et 80°, pour un angle d'incidence de la lumière de 45°.

Le trajet de couleur peut être mesuré par exemple au moyen d'un spectrogonioréflectomètre de marque INSTRUMENT SYSTEMS et de référence GON 360 GONIOMETER, après que la composition cosmétique ait été étalée à l'état fluide avec une épaisseur de 300 µm au moyen d'un étaleur automatique sur une carte de contraste de marque ERICHSEN et de référence Typ 24/5, la mesure étant effectuée sur le fond noir de la carte.
A titre d'exemple, on a représenté à la figure 1 un trajet de couleur obtenu avec un tel spectrogonioréflectomètre pour un gloss liquide réalisé conformément à l'invention, comportant des pigments g oniochromatiques SICOPEARL® commercialisés par la société BASF.
Un agent de coloration goniochromatique au sens de la présente invention permet d'observer un changement de couleur, encore appelé « color flop », en fonction de l'angle d'observation, supérieur à celui que l'on peut rencontrer avec des nacres.

Par « particules réfléchissantes », on désigne au sens de la présente invention des particules dont la taille, la structure, notamment l'épaisseur de la ou des couches qui la constituent et leur natures physique et chimique, et l'état de surface, leur permettent de réfléchir la lumière incidente avec une intensité suffisante pour pouvoir créer à la surface de la composition revendiquée, lorsque cette dernière est appliquée sur le support à maquiller, des points de surbrillance visibles à l'oeil nu, c'est-à-dire des points plus lumineux qui contrastent avec leur environnement en semblant briller.

Les particules réfléchissantes peuvent troubler la perception visuelle de la courbure du support maquillé, en tendant à empêcher une focalisation visuelle durable, les points de surbrillance étant susceptibles d'apparaître ou de disparaître de manière aléatoire lorsque le support maquillé et l'observateur sont animés.

Selon un mode mise en oeuvre, la brillance moyenne de la deuxième composition dépasse un certain seuil, notamment lorsque la composition est destinée à être appliquée sur les lèvres ou les ongles.

Par « brillance moyenne », on désigne la brillance telle qu'elle peut être mesurée à l'aide d'un brillancemètre, de manière conventionnelle, par la méthode suivante.
Sur une carte de contraste de marque LENETA et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER et de référence microTRI-GLOSS.

La brillance moyenne de la deuxième composition est avantageusement supérieure ou égale à 30, voire 50 ou mieux 70, notamment lorsque la composition est destinée à être appliquée sur les lèvres.

La composition peut comporter une base brillante destinée à permettre d'obtenir la brillance moyenne souhaitée.

Par « base », on désigne au sens de la présente invention la composition cosmétique sans le ou les agents colorants que la composition pourrait contenir.

La composition cosmétique peut ainsi comporter, dans un exemple de mise en oeuvre, une base dont la brillance moyenne est supérieure à 20, voire à 50 ou même à 70 notamment dans le cas d'une composition destinée à être appliquée sur les lèvres.

La présence d'agents de coloration tels que des pigments goniochromatiques ou des particules réfléchissantes dans la base brillante peut conduire à une composition cosmétique dont la brillance moyenne peut être différente ou non de la brillance moyenne de la base considérée isolément.

La formulation de la base pourra ainsi être différente selon que la composition cosmétique est destinée à former un gloss liquide ou un rouge à lèvres, par exemple. On pourra par exemple choisir une base de rouge à lèvres de brillance moyenne égale à 60 environ, une base de gloss liquide ou de fard à paupières de brillance moyenne égale à 70 environ, et une base de vernis à ongles de brillance moyenne égale à 50 environ.

La composition cosmétique peut comporter, notamment dans le cas où elle est destinée à être appliquée sur les lèvres, une phase huileuse, notamment une phase huileuse ayant un indice de réfraction compris entre 1,47 et 1,51, ce qui peut permettre d'obtenir une brillance relativement élevée.

Les particules réfléchissantes peuvent être présentes dans la composition en étant dispersées de manière homogène par exemple à une teneur allant de 0,1 % à 20 % par rapport au poids total de la composition, de préférence de 1 % à 15 % en poids, et mieux de 1 % à 10 % en poids, par exemple environ 2 % notamment pour une composition destinée à être appliquée sur les lèvres.
Selon un mode de réalisation particulier, les particules réfléchissantes peuvent être introduites de manière telle que le rapport pondéral particules réfléchissantes/pigments goniochromatiques varie de 0,3 à 3 et en particulier de 0,5 à 2,5. En fait, ce rapport peut varier en fonction de la nature de la composition cosmétique dans laquelle sont incorporées lesdites particules. Par exemple, dans une formulation de type vernis à ongles, ce rapport pondéral particules réfléchissantes/pigments goniochromatiques peut être supérieur à 1, en particulier supérieur à 1,5 et notamment supérieur ou égal à 2. En revanche, dans des formulations de type rouges à lèvres liquides ou en bâtonnets, ce rapport pondéral peut être inférieur ou égal à 2 notamment inférieur ou égal à 1,5.
Les p articules r éfléchissantes p euvent être o u non g oniochromatiques, i nterférentielles o u non, mais de préférence elles sont non goniochromatiques.
Les particules réfléchissantes présenteront de préférence une dimension d'au moins 10 µm, par exemple comprise entre environ 20 µm et environ 50 µm.
Par « dimension », on désigne la dimension donnée par la distribution granulométrique statistique à la moitié de la population, dite D50. La taille des particules réfléchissantes pourra dépendre de leur état de surface. Plus celui-ci est réfléchissant, plus la dimension pourra, a priori, être faible, et inversement.
Les particules réfléchissantes peuvent présenter des formes variées. Ces particules peuvent être notamment en forme de plaquettes ou globulaires, en particulier sphériques.
Par l'expression « en forme de plaquette », on désigne des particules dont le rapport de la plus grande dimension à l'épaisseur est supérieur ou égal à 5, voire 10 ou mieux 20. L'épaisseur des particules en forme de plaquettes est par exemple comprise entre environ 0,5 µm et environ 5 µm.
Les particules présentant une surface extérieure sensiblement plane conviennent tout particulièrement, car elles peuvent plus facilement donner naissance, si leur taille, leur structure et leur état de surface le permet, à une réflexion spéculaire intense. On parle d'effet miroir.
Il peut être souhaitable que les particules réfléchissantes soient non diffusantes et non mates.
Les particules réfléchissantes quelque soit leur forme, peuvent présenter une structure multicouche ou non et, dans le cas d'une structure multicouche, par exemple au moins une couche d'épaisseur uniforme, notamment d'un matériau réfléchissant.
Lorsque les particules réfléchissantes ne présentent pas de structure multicouche, elles peuvent être composées par exemple d'oxydes métalliques, par exemple des oxydes de titane ou de fer obtenus par synthèse de manière à présenter une surface sensiblement plane ayant un état de surface, par exemple non mat et non diffusant, permettant une réflexion spéculaire de la lumière suffisante pour obtenir des points de surbrillance au sein de la composition cosmétique.
Lorsque les particules réfléchissantes présentent une structure multicouche, celles-ci peuvent par exemple comporter un substrat naturel ou synthétique, notamment un substrat synthétique au moins partiellement enrobé par au moins une couche d'un matériau réfléchissant.
Quelque soit la forme des particules réfléchissantes, le substrat peut, lorsqu'il est synthétique, être réalisé avec une forme favorisant la formation d'une surface réfléchissante après revêtement, notamment après dépôt d'une couche de matériau réfléchissant. Le substrat peut, par exemple, présenter une surface plane et la couche de matériau réfléchissant une épaisseur sensiblement uniforme.
A titre d'exemple de particules réfléchissantes comportant un substrat minéral enrobé d'une couche de métal, on peut citer encore les particules comportant un substrat de borosilicate enrobé d'argent, encore appelées « nacres blanches ».
Des particules à substrat de verre revêtu d'argent, en forme de plaquettes, sont vendues sous la dénomination MICROGLASS METASHINE REFSX 2025 PS par la société TOYAL.

Des particules à substrat de verre revêtu d'alliage nickel/chrome/molybdène sont vendues sous la dénomination CRYSTAL STAR GF 550, GF 2525 par cette même société.
Les particules réfléchissantes quelque soit leur forme, peuvent également être choisies parmi les particules à substrat synthétique enrobé au moins partiellement d'au moins une couche d'au moins un composé métallique, notamment un oxyde métallique, choisi par exemple parmi les oxydes de titane, notamment TiO₂, de fer notamment Fe₂O₃, d'étain, de chrome, le sulfate de baryum et les composés suivants : MgF₂, CrF₃, ZnS, ZnSe, SiO₂, Al₂O₃, MgO, Y₂O₃, SeO₃, SiO, HfO₂, ZrO₂, CeO₂, Nb₂O₅, Ta₂O₅, MoS₂ et leurs mélanges ou alliages.
A titre d'exemple de telles particules, on peut citer par exemple les particules comportant un substrat de mica synthétique revêtu de dioxyde de titane, ou les particules de verre enrobé soit d'oxyde de fer brun, soit d'oxyde de titane, d'oxyde d'étain ou d'un de leurs mélanges comme celles vendues sous la marque REFLECKS® par la société ENGELHARD.
Conviennent également à l'invention, les pigments de la gamme METASHINE 1080R commercialisée par la Société NIPPON SHEET GLASS CO. LTD. Ces pigments, plus particulièrement décrits dans la demande de brevet JP 2001-11340, sont des paillettes de verre C-GLASS comprenant 65 à 72 % de SiO₂, recouvertes d'une couche d'oxyde de titane de type rutile (TiO₂). Ces paillettes de verre ont une épaisseur moyenne de 1 micron et une taille moyenne de 80 microns soit un rapport en taille moyenne/épaisseur moyenne de 80. Elles présentent des reflets bleus, verts, jaunes ou de teinte argent selon l'épaisseur de la couche de TiO₂.
On peut encore citer les particules de dimension comprise entre 80 et 100 µm, comportant un substrat de mica synthétique (fluorophlogopite) revêtu de dioxyde de titane représentant 12% du poids total de la particule, vendues sous la dénomination PROMINENCE par la société NIHON KOKEN.
Les particules réfléchissantes peuvent encore être choisies parmi les particules formées d'un empilement d'au moins deux couches à indices de réfraction différents.
Ces couches peuvent être de nature polymérique ou métallique et notamment inclure au moins une couche polymérique.
Ainsi, les particules réfléchissantes peuvent être des particules dérivant d'un film polymérique multicouche.
De telles particules sont notamment décrites dans WO 99/36477, US 6 299 979 et US 6 387 498.
Des particules réfléchissantes comportant un empilement d'au moins deux couches de polymères sont commercialisées par la société 3M sous la dénomination MIRROR GLITTER. Ces particules comportent des couches de 2,6-PEN et de polyméthacrylate de méthyle dans un rapport massique de 80/20. De telles particules sont décrites dans le brevet US 5 825 643.

La deuxième cosmétique selon l'invention peut bien entendu comporter des particules réfléchissantes de différentes natures sans que l'on sorte du cadre de la présente invention.

### Exemples d'agents de coloration goniochromatiques

La composition contient un ou plusieurs agents de coloration goniochromatiques pour créer, lorsque la composition est appliquée sur son support un fond coloré dont la couleur change avec l'angle d'observation et avec lequel les particules réfléchissantes contrastent. On peut utiliser un seul agent de coloration goniochromatique pour une facilité de mise en oeuvre.

L'agent de coloration goniochromatique peut être présent par exemple en une quantité pouvant varier, en poids par rapport au poids total de la composition, de 0,1 à 20 % ou de 2 à 15 %, et mieux de 2 à 10 % notamment pour une composition destinée à être appliquée sur les lèvres. Dans le cas d'une telle composition, des résultats très satisfaisants ont pu être obtenus pour une teneur d'agent de coloration goniochromatique comprise entre 2 et 8 % combinée à une teneur en particules réfléchissantes comprise entre 1 et 5 %, en poids. Une composition de vernis à ongles pourra contenir par exemple de 0,1 à 5% d'agent de coloration goniochromatique; un fond de teint pourra en contenir de 10 à 15% et un rouge à lèvres pourra en contenir de 2 à 8 % en poids.
L'agent de coloration goniochromatique peut être choisi de manière à présenter un changement de couleur relativement important avec l'angle d'observation.
L'agent de coloration goniochromatique peut ainsi être choisi de telle sorte que l'on puisse observer, pour une variation de l'angle d'observation comprise entre 0° et 80° sous un éclairage à 45°, une variation de couleur ΔE de la composition cosmétique, mesurée dans l'espace colorimétrique CIE 1976, d'au moins 2.
L'agent de coloration goniochromatique peut également être choisi de telle sorte que l'on puisse observer, pour un éclairage à 45° et une variation de l'angle d'observation comprise entre 0° et 80°, une variation Dh de l'angle de teinte de la composition cosmétique, dans le plan CIE 1976, d'au moins 30° voire au moins 40° ou au moins 60°, voire encore d'au moins 100°.
L'agent de coloration g oniochromatique peut être choisi par exemple parmi les structures multicouche interférentielles et les agents de coloration à cristaux liquides.
Dans le cas d'une structure multicouche, celle-ci peut comporter par exemple au moins deux couches, chaque couche, indépendamment ou non de la (ou les) autre(s) couche(s), étant réalisée par exemple à partir d'au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.
La structure multicouche peut présenter ou non, par rapport à une couche centrale, une symétrie au niveau de la nature chimique des couches empilées.
Des exemples des tructures multicouche interférentielles s ymétriques utilisables d ans d es compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MoS₂/SiO₂/Al/SiO₂/MoS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂/MoS₂ ; Fe₂O₃/SiO₂/mica-oxyde/SiO₂/Fe₂O₃ ; TiO₂/SiO₂/TiO₂ et TiO₂/Al₂O₃/TiO₂, des pigments ayant ces structures étant commercialisés sous la dénomination XIRONA par la société MERCK (Darmstadt). On peut encore citer les pigments INFINITE C OLORS d e la société S HISEIDO. Selon l'épaisseur et la n ature d es différentes couches, on obtient différents effets. Ainsi, avec la structure Fe₂O₃/SiO₂/Al/SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.
Des agents de coloration goniochromatiques convenant également à l'invention sont des pigments de structure silice/oxyde de titane/oxyde d'étain commercialisés sous le nom XIRONA MAGIC par la Société MERCK, des pigments de structure silice/oxyde de zinc commercialisés sous le nom XIRONA INDIAN SUMMER par la Société MERCK et des pigments de structure silice/oxyde de titane/mica/oxyde d'étain commercialisés sous le nom XIRONA CARRIBEAN BLUE par la Société MERCK.
On peut encore utiliser des agents de coloration goniochromatiques à structure multicouche comprenant une alternance de couches polymériques par exemple du type naphtalate de polyéthylène et téréphtalate de polyéthylène. De tels agents sont notamment décrits dans WO-A-96/19347 et WO-A-99/36478.
On peut citer, à titre d'exemple de pigments à structure multicouche polymérique, ceux commercialisés par la société 3M sous la dénomination COLOR GLITTER.
Les agents de coloration à cristaux liquides comprennent par exemple des silicones ou des éthers de cellulose sur lesquels sont greffés des groupes mésomorphes.

Comme particules goniochromatiques à cristaux liquides, on peut utiliser par exemple celles vendues par la société CHENIX ainsi que celle commercialisées sous la dénomination HELICONE® HC par la société WACKER.
La composition peut en outre comporter des fibres goniochromatiques dispersées. De telles fibres peuvent par exemple présenter une taille comprise entre 200 µm et 700 µm, par exemple d'environ 300 µm.
En particulier, on peut utiliser des fibres interférentielles à structure multicouche. Des fibres à structure multicouche de polymères sont notamment décrites dans les documents EP-A-921217, EP-A-686858 et US-A-5472798. La structure multicouche peut comporter au moins deux couches, chaque couche, indépendamment ou non de la (ou les) autre(s) couche(s), étant réalisée en au moins un polymère de synthèse. Les polymères présents dans les fibres peuvent avoir un indice de réfraction allant de 1,30 à 1,82 et mieux allant de 1,35 à 1,75. Les polymères préférés pour constituer les fibres sont les polyesters tels que le polyéthylène téréphtalate, le polyéthylène naphtalate, le polycarbonate ; les polymères acryliques comme le polyméthacrylate de méthyle ; les polyamides.
Des fibres goniochromatiques à structure bicouche polyéthylène téréphtalate/nylon-6 sont commercialisées par la société TEIJIN sous la dénomination MORPHOTEX.

### Base brillante

La deuxième composition peut également comprendre au moins un composé capable de lui conférer de la brillance et notamment une phase huileuse, en particulier une phase huileuse présentant un indice de réfraction compris entre 1,47 et 1,51, mieux entre 1,48 et 1,50.

L'indice de réfraction est mesuré à température ambiante (25°C), à l'aide d'un réfractomètre. Une telle phase huileuse peut s'avérer utile dans le cas d'un gloss liquide notamment.
Selon un exemple de mise en oeuvre de l'invention, on choisit une base brillante telle que décrite dans la demande EP-A-792 637, dont le contenu est incorporé par référence dans la présente demande.

La composition cosmétique peut contenir par exemple au moins une huile d'origine minérale, végétale ou synthétique, carbonée, hydrocarbonée, fluorée et/ou siliconée.

### Additifs

Les première et/ou seconde compositions du produit cosmétique selon l'invention peuvent également contenir un ou plusieurs actifs cosmétiques, dermatologiques, hygiéniques ou pharmaceutiques.

Comme actifs cosmétiques, dermatologiques, hygiéniques ou pharmaceutiques, utilisables dans les compositions de l'invention, on peut citer les hydratants (polyol comme glycérine), vitamines( C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...).

Ces actifs sont utilisés en quantité habituelle pour l'homme de l'art et notamment à des concentrations de 0 à 20 % et notamment de 0,001 à 15 % du poids total de la première et/ou seconde composition.

Chaque composition du produit selon l'invention peut, de plus, comprendre, selon le type d'application envisagée, les constituants classiquement utilisés dans les domaines considérés, qui sont présents en une quantité appropriée à la forme galénique souhaitée.

De manière générale, les milieux physiologiquement acceptables de chacune des première et seconde compositions du produit selon l'invention peuvent comprendre, outre les huiles, des corps gras additionnels qui peuvent être choisis parmi les cires, les huiles, les gommes et/ou les corps gras pâteux.

De préférence, le milieu physiologiquement acceptable de la première et/ou de la seconde composition contient un corps gras pâteux et/ou une cire choisie parmi les cires précédemment citées.

Chaque composition du produit selon l'invention peuvent contenir, en outre, tout additif usuellement utilisé dans de telles compositions, comme des épaississants d'huile ou de phase aqueuse (gélifiant acrylique), des antioxydants, des parfums, des conservateurs (pentylène glycol), des tensioactifs, des polymères liposolubles (copolymère de polyvinylpyrrolidone/eicosène par exemple).

Lorsque le milieu physiologiquement acceptable de la première et/ou de la seconde composition contient une phase organique liquide, ce milieu peut notamment contenir de l'eau dispersée ou émulsionnée dans ladite phase organique liquide.

Dans un mode de réalisation particulier de l'invention, les compositions selon l'invention peuvent être préparées de manière usuelle par l'homme du métier. Elles peuvent se présenter sous forme d'un produit coulé et par exemple sous la forme d'un stick ou bâton, sous forme de pâte souple dans une bouillotte, ou sous la forme de coupelle utilisable par contact direct ou à l'éponge. En particulier, elles constituent, ensemble ou séparément, un fond de teint coulé, fard à joues ou à paupières coulé notamment coloré, rouge à lèvres, brillant pour les lèvres, produit anti-cernes. Elles peuvent aussi se présenter chacune sous forme d'une pâte souple ou encore de gel, de crème plus ou moins fluide. Elles peuvent alors constituer des fonds de teint ou des rouges à lèvres fluides ou pâteux, des brillants à lèvres, des produits solaires ou de coloration de la peau, des eyeliners, des produits de maquillage du corps ou encore présenter des propriétés de soin et se présenter alors sous forme de base ou de baume de soin des lèvres.

Chaque composition du produit selon l'invention peuvent se présenter sous toute forme galénique normalement utilisée pour une application topique et notamment sous forme d'une solution huileuse ou aqueuse, d'un gel huileux ou aqueux, d'une émulsion huile-dans-eau ou eau-dans-huile, d'une émulsion multiple, d'une dispersion d'huile dans de l'eau grâce à des vésicules, les vésicules étant situés à l'interface huile/eau, ou d'une poudre. Chaque composition peut être fluide ou solide.

Avantageusement, la première ou la seconde composition, ou les deux, sont à phase grasse continue et de préférence sous forme anhydre et peuvent contenir moins de 5% d'eau, et encore mieux moins de 1% d'eau par rapport au poids total de la première ou seconde composition. En particulier, le produit de maquillage bicouche entier est sous forme anhydre.

Chaque première et seconde composition peut avoir l'aspect d'une lotion, d'une crème, d'une pommade, d'une pâte souple, d'un onguent, d'un solide coulé ou moulé et notamment en stick ou en coupelle, ou encore de solide compacté.

Chaque composition peut être conditionnée séparément dans un même article de conditionnement par exemple dans un stylo bi-compartimenté, la composition de base étant délivrée par une extrémité du stylo et la composition du dessus étant délivrée par l'autre extrémité du stylo, chaque extrémité étant fermée notamment de façon étanche par un capuchon.

De préférence, la composition qui est appliquée en première couche est sous forme solide, ce qui permet une application plus pratique, une meilleure stabilité dans le temps et en température de la composition et permet un tracé précis du maquillage, ce qui est hautement souhaitable dans le cas d'un rouge à lèvres ou d'un eyeliner.

Le produit selon l'invention peut être avantageusement utilisé pour le maquillage de la peau et/ou des lèvres et/ou des phanères selon la nature des ingrédients utilisés. En particulier, le produit de l'invention peut être se présenter sous forme, de fond de teint solide, de bâton ou pâte de rouge à lèvres, de produit anti-cernes, ou contours des yeux, d'eye liner, de mascara, d'ombre à paupières, d'un vernis à ongles, de produit de maquillage du corps ou encore un produit de coloration de la peau.

Le produit est en particulier un rouge à lèvres.

De préférence, la première et/ou la seconde composition est sous forme solide.

Avantageusement, la couche du dessus présente des propriétés de soin et/ou de brillance.

L'invention a encore pour objet un produit à lèvres, un fond de teint, un tatouage, un fard à joues ou à paupières contenant une première et une seconde compositions telles que décrites précédemment.

Les compositions de l'invention peuvent être obtenues par chauffage des différents constituants à la température de fusion des cires les plus élevées, puis coulage du mélange fondu dans un moule ( coupelle ou doigt de gant). Elles peuvent aussi être obtenues par extrusion comme décrit dans la demande EP-A-0667 146.

Les exemples ci-dessous ont pour but d'illustrer de manière non limitative la présente invention.

Les quantités sont données en pourcentages massiques .

### EXEMPLE 1 : Composition lissante pour les lèvres

| | |
|---|---|
| Polyisobutène hydrogéné (Nof Corp.) | 10 % |
| propionate d'arachidyle (Alzo) | 1,8 % |
| néopentanoate d'octydodécyle (Bernel) | 1,3 % |
| disteardinonium hectorite (Elementis) | 0,3 % |
| mica recouvert de microbilles d'hydroxyde d'aluminium (Excel Mica JP-2 ® de Miyoshi) | 5 % |
| amidon réticulé par l'anhydride octényl succinique (Dry Flo Plus (28-1160) ® de National Starch) | 10% |
| polybutène | 4,7 % |
| cire de polyéthylène | 15,8 % |
| diméthicone (DC 200-ScSt) | 17,3 % |
| décaméthyl tétrasiloxane (DC 200-1,5 cSt) | 22 % |
| octyldodécanol | 3,2 % |
| cire de lanoline oxypropylénée (50P) (Cognis) | 8,4 % |

### Best mode

### EXEMPLE 2 : Rouge à lèvres liquide brillant

| | |
|---|---|
| Polyacryladipate-2 de bis-diglycéryle | 17,5 % |
| Malate de diisostéraryle | 9,5 % |
| Trimellitate de tridécyle | 10 % |
| Triglycéride d'acide C18-36 | 19 % |
| Silice diméthyl silylate | 8 % |
| Particules de verre enrobé d'argent (METASHINE®)* | 2 % |
| Pigment goniochromatique (SICOPEARL®)** | 5 % |
| Nacre | 3 % |
| Polybutène | 12 % |
| Tétraisostéarate de pentaerythrityle | 13 % |
| Parfum, conservateur | qs |

| | |
|---|---|
| * commercialisées par la société TOYAL | |
| ** commercialisé par la société BASF | |

Le rouge à lèvres liquide brillant peut être avantageusement appliqué sur la composition lissante de l'exemple 1.

## Revendications

1. Produit cosmétique de maquillage et/ou de soin contenant une première et une seconde composition, la première composition comprenant un premier milieu physiologiquement acceptable et présentant un indice de flou (Th-Td)/Th*100 supérieur ou égal à 40%, et un indice de transparence Th supérieur ou égal à 70%, et la seconde composition comprenant un second milieu physiologiquement acceptable.

2. Produit selon la revendication 1, **caractérisé en ce que** l'indice de flou est supérieur ou égal à 50%.

3. Produit selon la revendication 1 ou 2, **caractérisé en ce que** l'indice de transparence est supérieur ou égal à 85%, de préférence supérieur ou égal à 95%.

4. Produit selon l'une des revendications précédentes, **caractérisé en ce que** la première composition comprend un composé minéral ou organique en quantité suffisante pour que ladite composition présente un indice de flou supérieur ou égal à 40%, et un indice de transparence supérieur ou égal à 70%.

5. Produit selon l'une des revendications précédentes, **caractérisé en ce que** la première composition comprend un composé choisi parmi les charges, les organosiloxanes et les polymères non siliconés de faible masse moléculaire.

6. Produit selon la revendication 5, **caractérisé en ce que** la granulométrie moyenne des charges est inférieure ou égale à 50 microns, de préférence 30 microns, de préférence encore 15 microns, et préférentiellement inférieure ou égale 10 microns, par exemple de l'ordre de 5 ou 3 microns.

7. Produit selon la revendication 5 ou 6, **caractérisé en ce que** la charge est choisie parmi les charges d'origine naturelle ou synthétique, notamment le talc, l'amidon, le mica, la silice, les poudres de nylon, les poudres de polyéthylène, la poly-bêta-alanine, les poly(méthacrylate de méthyle) et leurs mélanges.

8. Produit selon la revendication 5, **caractérisé en ce que** l'organopolysiloxane est choisi parmi:
i) les polyorganopolysiloxanes comprenant des motifs R₂SiO et R SiO_{1,5} et éventuellement des motifs R₃SiO_{0,5} et/ou SiO₂ dans lesquels les radicaux R, indépendamment les uns des autres, désignent un hydrogène, un alkyle tel que méthyle, éthyle ou propyle, un aryle tel que phényle ou tolyle, un groupe aliphatique insaturé tel q ue vinyle et où le rapport en poids des motifs R₂SiO sur les motifs RSiO_{1,5} varie de 1/1 à 30/1 ;
ii) les polyorganopolysiloxanes insolubles et gonflables dans l'huile de silicone, obtenus par addition d'un organohydrogénopolysiloxane (1) et d'un organopolysiloxane (2) ayant des groupes aliphatiques insaturés de telle sorte que la quantité d'hydrogène ou de groupes aliphatiques insaturés dans respectivement (1) et (2) soit comprise entre 1 et 20% mol quand l'organopolysiloxane est non-cyclique et entre 1 et 50% mol lorsque l'organopolysiloxane est cyclique.

9. Produit selon la revendication 5, **caractérisé en ce que** les polymères non siliconés de faible masse moléculaire sont notamment des polymères amide, uréthanne ou urée de masse moléculaire moyenne en poids comprise entre 200 et 1000 g/mol.

10. Produit selon la revendication 9, caractérisé en ce les polymères ont des chaînes pendantes et/ou terminales alkyles ou alkylènes, comprenant de 8 à 60 atomes de carbone, de préférence de 12 à 40 atomes de carbone.

11. Produit selon l'une quelconque des revendications précédentes, **caractérisé en ce que** la première composition comprend une charge minérale et une charge organique.

12. Produit selon la revendication précédente, **caractérisé en ce que** la charge minérale est lamellaire lorsque la charge organique est sphérique et vice versa.

13. Produit selon la revendication 11, **caractérisé en ce que** la charge minérale est constituée ou comprend au moins un matériau choisi par exemple parmi le talc, le mica, la s ilice, le kaolin, les microsphères de silice creuses, les m icrocapsules de verre, les oxydes de titane, les oxydes de fer, les oxydes de zinc, les oxydes d'aluminium, le sulfate de baryum, les hydroxydes métalliques et leurs mélanges.

14. Produit selon la revendication 11, **caractérisé en ce que** la charge organique est choisie parmi la poudre de polyéthylène, la poudre d'amidon, la poudre d'amidon modifiée comme l'amidon réticulé par l'anhydride octénylsuccinique, la poudre de nylon, les poudres de copolymères styrène/acrylique et leurs mélanges.

15. Produit selon l'une quelconque des revendications précédentes, **caractérisé en ce que** la première composition comprend une charge minérale comprenant du mica, ladite charge ayant une taille moyenne des particules inférieure ou égale à 50, de préférence inférieure à 10 microns, de préférence encore inférieure à 5 microns.

16. Produit selon l'une quelconque des revendications précédentes, **caractérisé en ce que** la première composition comprend une charge organique comprenant de l'amidon, ladite charge ayant une taille moyenne des particules inférieure ou égale à 50, de préférence inférieure à 10 microns, de préférence encore inférieure à 5 microns.

17. Produit selon la revendication 5, **caractérisé en ce que** le composé représente de 0,1 à 40%, de préférence encore de 3% à 15%, en poids par rapport au poids total de la composition.

18. Produit selon l'une quelconque des revendications précédentes, **caractérisé en ce que** la première composition comprend une huile volatile.

19. Produit selon l'une quelconque des revendications précédentes, **caractérisé par le fait que** la brillance moyenne de la deuxième composition est supérieure ou égale à 30, de préférence supérieure ou égale à 50, de préférence supérieure ou égale à 70.

20. Produit selon la revendication précédente, **caractérisé par le fait que** la deuxième composition comporte, dans un milieu physiologiquement acceptable, au moins un agent de coloration goniochromatique ou des particules réfléchissantes.

21. Produit selon l'une des revendications précédentes, **caractérisé en ce qu'**il se présente sous la forme d'un fond de teint, d'un fard à joues ou à paupières, d'un rouge à lèvres, d'un produit ayant des propriétés de soin, d'un eye-liner, d'un produit anti-cernes, ou d'un produit de maquillage du corps.

22. Produit selon l'une des revendications précédentes, **caractérisé en ce que** la première ou la seconde composition, ou les deux, sont sous forme anhydre.

23. Produit selon l'une quelconque des revendications précédentes, **caractérisé par le fait que** la deuxième composition se présente sous la forme d'un rouge à lèvres en stick ou d'un gloss liquide.

24. Procédé de maquillage de la peau, des lèvres ou des phanères consistant à appliquer sur la peau, les lèvres et/ou les phanères une couche d'une première composition comprenant un premier milieu physiologiquement acceptable et contenant au moins une charge lui conférant un indice de flou (Th-Td)/Th*100 supérieur ou égal à 40%, et un indice de transparence Th supérieur ou égal à 70%, et une couche d'une seconde composition comprenant un second milieu physiologiquement acceptable, la couche de ladite première composition étant appliquée préalablement à la couche de ladite seconde composition, ou inversement.

25. Procédé de maquillage de la peau, des lèvres ou des phanères consistant à appliquer sur la peau et/ou les lèvres et/ou les phanères, dans un ordre ou dans l'autre, les première et seconde compositions d'un produit cosmétique de maquillage selon l'une des revendications 1 à 23.

26. Kit de maquillage comprenant un produit selon l'une des revendications 1 à 23.

27. Utilisation cosmétique du produit selon l'une des revendications 1 à 23 pour améliorer l'apparence de la surface de la peau, des lèvres ou des phanères et/ou pour modifier la perception du volume de la partie du corps sur laquelle il est appliqué, pour obtenir un dépôt brillant, de bonne tenue, non migrant, confortable, de bonne tenue de la couleur et/ou de bonne tenue de la brillance.
